Europäisches Patentamt

**European Patent Office**

Office européen des brevets

(11) Veröffentlichungsnummer: **0 001 074**
**B1**

(12) **EUROPÄISCHE PATENTSCHRIFT**

(45) Veröffentlichungstag der Patentschrift:
02.06.82

(21) Anmeldenummer: **78100735.6**

(22) Anmeldetag: **24.08.78**

(51) Int. Cl.³: **A 61 M 1/03**

(54) **Gerät zur Behandlung urämischer Patienten.**

(30) Priorität: **01.09.77 DE 2739350**

(43) Veröffentlichungstag der Anmeldung:
**21.03.79 Patentblatt 79/6**

(45) Bekanntmachung des Hinweises auf die Patenterteilung:
**02.06.82 Patentblatt 82/22**

(84) Benannte Vertragsstaaten:
**BE CH DE FR GB LU NL SE**

(56) Entgegenhaltungen:
**BE-A-832 648**
**BE-A-834 361**
**DE-A-2 101 168**
**DE-A-2 149 040**
**DE-A-2 218 524**
**DE-A-2 518 108**
**DE-A-2 557 604**
**DG-B-2 101 168**
**FR-A-2 344 297**
**FR-A-2 345 165**
**US-A-2 152 355**
**US-A-3 228 397**
**US-A-3 557 789**
**US-A-4 041 944**

(73) Patentinhaber: **SCHI - WA Arzneimittelwerk GmbH,
D-4518 Bad Laer-Glandorf (DE)**

(72) Erfinder: **Kramer, Peter, Dr., Gutenbergstrasse 21,
D-3400 Goettingen (DE)**

(74) Vertreter: **Bokelmann, Ekkehart, Dr. et al, i.Fa. BASF
Aktiengesellschaft Carl-Bosch-Strasse 38,
D-6700 Ludwigshafen (DE)**

**Klinische Wochenschrift, 55,
1121 (1977)**

## Gerät zur Behandlung urämischer Patienten

Bei urämischen Patienten muß Plasmawasser, das harnpflichtige Substanzen bzw. urämische Toxine enthält, mittels Ultrafiltration durch ein Hämofilter nach Heparinisierung vom Blut abgetrennt und gegebenenfalls nach Entfernung eines der Urinausscheidung entsprechenden Anteils durch eine Substitutionslösung ersetzt werden, der vor der Einführung in den Organismus das entwässerte Restblut zugemischt wird (Klin. Wschr., 55, 1121 (1977).

Zur Beschleunigung dieses Vorganges soll die Filtration durch einen Unterdruck in der Größenordnung von 40 mm Hg auf der Seite des Plasmawassers und durch einen etwa gleichgroßen Druck auf das Restblut/Substitutionslösungs-Gemisch unterstützt werden (Klin. Wschr., 55, 1121 (1977). Außerdem muß sicher gewährleistet sein, daß weder eine Überwässerung noch eine übermäßige Entwässerung des Patienten erfolgen kann, sondern daß die therapeutisch vorgesehenen Flüssigkeitsmengen genau und die dafür vorgesehenen Zeiten annähernd genau eingehalten werden. Die bisher für diesen Zweck eingesetzten »künstlichen Nieren« sind sehr teuer und bergen durch ihre komplizierte Technik Sicherheitsrisiken in sich, die durch Erschöpfung der für sie benötigten Energiequellen während der Behandlung durch Ausfall oder Abnutzung elektromechanischer Steuerungselemente sowie durch Schädigung der Blutzellen auftreten können. Weiter besteht durch die Benutzung von Blutpumpen die Gefahr von Luftembolien.

Aus der US-A-4 041 944 ist eine Blutaustauschvorrichtung bekannt, mit deren Hilfe eine bestimmte Menge Blut gegen das gleiche Volumen einer Ersatzflüssigkeit nach dem Volumenaustauschprinzip ersetzt wird. Die Ersatzflüssigkeit wird zu diesem Zweck in einem Beutel angeordnet, der in einem geschlossenen, flüssigkeitsgefüllten Behälter angeordnet ist, in den das entnommene Blut eingeleitet wird. Hierfür wird jedoch eine Pumpe benötigt, wodurch die Vorrichtung störanfällig wird und die Gefahr besteht, daß Luftembolien auftreten.

Weiterhin sind Flüssigkeitsaustauschvorrichtungen für die Peritonealdialyse beschrieben worden (DE-A-2 149 040 und DE-B-2 101 168). Bei Geräten der in der DE-B-2 101 168 beschriebenen Art werden die Volumen der ein- und auslaufenden Spüllösung durch gegenseitige Verdrängung mit Hilfe von zwei Beuteln, die in einem oder in zwei miteinander verbundenen flüssigkeitsgefüllten Behältern angeordnet sind, miteinander verglichen und deren Füllzustand zur Steuerung der Pumpen herangezogen. Diese Vorrichtung entspricht in ihrem Aufbau weitgehend der in der US-A-4 041 944 geschilderten Blutaustauschvorrichtung hinsichtlich des Volumenaustauschprinzips. Auch hier sind störanfällige Pumpen erforderlich. Für sie gelten daher die geschilderten Nachteile in gleicher Weise.

Bei Vorrichtungen nach der DE-A-2 149 040 ist zur Gewährleistung der Zuführung einer gleichen Menge einer Dialysierflüssigkeit zur Bauchhöhle, wie sie der Bauchhöhle abgezogen wird, ein durch einen mit Ventilklappen versehenen Membrankolben in zwei Räume getrennter Behälter vorgesehen. Der Membrankolben ist zur Erzielung einer Druckdifferenz federbelastet. Dieses Gerät ist zwar unabhängig von elektrisch betriebenen Pumpen. Jedoch ändert sich in Abhängigkeit von der Stellung des Membrankolbens, d. h. in Abhängigkeit vom Federweg die Federkraft und damit in unerwünschter Weise auch der Förderdruck dieser Pumpe.

Bei Dialysiervorrichtungen nach der DE-A-2 152 355 wird das frisch entnommene Blut über einen Hämofilter in Plasmawasser und Restblut getrennt. das Plasmawasser wird anschließend durch eine Aufbereitungsanlage geführt und zusammen mit dem Restblut wieder dem menschlichen Körper zugeführt. Der für den Transport des Plasmawassers nötige Druck wird durch eine Pumpe erzeugt, wodurch die vorstehend bereits angeführten Nachteile eintreten können.

Die vorliegende Erfindung betrifft ein Gerät zur Behandlung urämischer Patienten, welches diese Nachteile nicht besitzt, und bei dem das frisch entnommene Blut über ein Hämofilter (F) in Plasmawasser und Restblut getrennt und das Plasmawasser anschließend in eine Austauschvorrichtung geleitet wird, aus dem eine Substitutionslösung nach Zusammenführen mit dem Restblut in den Blutkreislauf zurückgeführt wird, wobei die Austauschvorrichtung aus einem vollständig mit Flüssigkeit gefüllten luftdicht verschließbaren System (A; I, II, III) besteht, welches aus zwei durch eine flexible oder bewegliche Trennfläche (m) voneinander getrennten Kammern (A, B; I, II) besteht, von denen die erste Kammer (A; I) mit Flüssigkeit gefüllt ist und zusätzlich das Plasmawasser aufnehmen kann und die zweite Kammer (B; II) die Substitutionslösung enthält, dadurch gekennzeichnet, daß durch Gewichtsdruck (D; g) auf die flexible oder bewegliche Trennfläche (m) in der ersten Kammer (A; I) ein Unterdruck und in der zweiten Kammer (B; II) ein Überdruck erzeugt wird.

In die erste Kammer (A; I) fließt das durch Hämofiltration gewonnene Plasmawasser und verdrängt nach dem Volumenverschiebungsprinzip aus der zweiten Kammer (B; II) ein identisches Volumen von dort befindlicher Substitutionslösung. Diese Substitutionslösung wird mit der nach Passieren des Hämofilters (F) verbleibenden Restblutmenge vermischt und einer Körpervene zugeführt.

Die Trennwand (m) zwischen der ersten und der zweiten Kammer ist verschiebbar oder verformbar gestaltet, und zwar so, daß auf diese Trennfläche wirkende Gewichte in der ersten Kammer (A; I) einen den Filtrationsprozeß fördernden Unterdruck und gleichzeitig in der zweiten Kammer (B; II) einen die Rückführung der Substitutionslösung fördernden Überdruck erzeugen.

Das Plasmawasser kann entweder in die bereits vollständig mit Flüssigkeit gefüllte, dicht abgeschlossene erste Kammer (A; I) oder zweckmäßigerweise in einen dort befindlichen Beutel (p) geleitet werden. Die Substitutionslösung in der zweiten Kammer (B; II bzw. III) befindet sich vorzugsweise in einem oder mehreren untereinander verbundenen Beuteln (B, s).

Es ist auch möglich, den Druck aus der zweiten Kammer (II) in eine Nebenkammer (III) zu übertragen, in der sich die Substitutionslösung befindet.

Die zweite Kammer kann auch durch einen oder mehrere Beutel (B) dargestellt werden, die in der ersten Kammer liegen. Allerdings muß dann der Druck, der normalerweise in der zweiten Kammer herrscht, direkt auf den oder die Beutel übertragen werden.

Die Menge der im Austausch gegen das Plasmawasser zugeführten Substitutionslösung kann zur therapeutischen Entwässerung reduziert werden. Das kann erreicht werden durch Ableiten entsprechender Mengen von Plasmawasser vor Eintritt in die erste Kammer (A; I) oder von Flüssigkeit aus der zweiten Kammer (II). Dazu kann ein Beutel (O) oder eine weitere Kammer (IV) verwendet werden, die außerhalb der ersten und zweiten Kammer (A; I, II) angeordnet sind und über ein Ventil (w) mit der Leitung des Hämofiltrats oder mit der zweiten Kammer (II) verbunden sind.

Die neue Vorrichtung ist wesentlich billiger als die bislang bekannten Dialysegeräte. Außerdem bietet sie einen hohen Grad von Sicherheit (durch den Verzicht auf eine Blutpumpe wird die Gefahr einer Luftembolie für den Patienten stark verringert), so daß sie zu Hause eingesetzt werden kann. Schließlich ist das Gerät von Wasser- und Stromnetz unabhängig.

### Figur 1

In einen Kasten A, der luftdicht mit einem Deckel verschließbar ist, werden zwei 5-Liter-Beutel mit Substitutionslösung B gelegt und die Anschlußstutzen durch besondere Abdichtungen C in einer der Seitenwände nach außen durchgeführt. Die Anschlußschläuche, die mit dem Restblut-führenden Schlauch hinter dem Hämofilter F verbunden sind, werden mit Lösung gefüllt und dann abgeklemmt (Klemme N). Auf die Infusionsbeutel wird der Druck der Gewichte übertragen (Gewicht 20 bis 40 kg). Dadurch entsteht beispielsweise in den Infusionsbeuteln ein Überdruck von 40 bis 80 mm Hg. Der Behälter wird luftblasenfrei bis zum Rand mit Wasser gefüllt und mit dem Deckel E verschlossen. Die restliche Luft, die sich in der Wölbung des Deckels gesammelt hat, wird durch Öffnung der Zusatzinfusion G, die über die Tropfkammer und den Ultrafiltrationseinlaß H in den Behälter gelangt, durch das Entlüftungsventil I ausgetrieben und dieses anschließend verschlossen.

Aus diesem geschlossenen Behälter wird nun nur so viel Substitutionslösung durch den Druck der Gewichte getrieben, wie Ultrafiltrat in den Behälter nachfließt. Die Gewichte erzeugen nicht nur einen Überdruck in den Infusionsbeuteln, sondern auch einen Unterdruck in der die Infusionsbeutel umgebenden Flüssigkeit. Dieser Unterdruck fördert nach Anschluß des Gerätes an den Patienten den Einstrom von Ultrafiltrat bzw. Plasmawasser. — Das Blut wird unter Ausnutzung des arterio-venösen Druckgradienten (»mit Herzkraft«) durch das Hämofilter getrieben, wobei der durch die Gewichte D erzeugte Unterdruck die Filtration fördert. Blutverluste durch eine Membranruptur sollen durch einen Trübungsmesser K im Ultrafiltrat-führenden Schlauch frühzeitig Alarm auslösen. Die Ultrafiltrationsrate kann in der Tropfkammer H anhand der Tropfgeschwindigkeit geschätzt werden.

Nachdem zu Beginn der Behandlung die Zusatzinfusion abgeklemmt (Klemme L) und der Ultrafiltrat- sowie der Substitutionslösung-führende Schlauch geöffnet sind (Klemmen M und N), wird die Menge des ultrafiltrierten Blutwassers quantitativ durch Substitutionslösung ersetzt. Ein effektiver Flüssigkeitsentzug wird durch Abzweigung eines Teiles des Ultrafiltrats in den luftleeren Sammelbeutel O errreicht. Durch Aufhängen des Sammelbeutels unterhalb des Patienten wird hydrostatisch ein geringer Unterdruck erzeugt. Der Ultrafiltrationsstrom wird nun durch ein alternierendes Quetschventil Q mit einem stufenlos variablen elektronischen Intervallschalter entweder in Richtung Substitutionsbehälter A oder Sammelbeutel O freigegeben. Durch Wahl der Zeitintervalle (z. B. 48 sec → Substitutionsbehälter/12 sec → Sammelbeutel) kann ein quasi-kontinuierlicher Flüssigkeitsentzug erzielt werden. Bei einer Filtrationsrate von 10 ml/min verliert dabei der Patient 2 ml/min bzw. 120 ml/h. Ein übermäßiger Flüssigkeitsentzug wird durch das begrenzte Füllungsvolumen des Sammelbeutels O (1,5 bis 2,5 Liter) verhindert. Eine übermäßige Flüssigkeitszufuhr durch Eindringen von Luft in den Behälter A wird durch eine Flüssigkeitsstandüberwachung P (Licht-Glas-Reflexionssonde) verhindert. Wenn der Glaskegel dieser Sonde den Kontakt mit der Flüssigkeit verliert, wird ein Alarm ausgelöst.

Mit der Heparinpumpe R kann Heparin der Blutflüssigkeit zugesetzt werden. Die Heparinpumpe kann ebenso wie der Trübungsmesser K und das Quetschventil Q und die Flüssigkeitsstandüberwachung P mit Hilfe von Batterien betrieben werden.

Figur 2

Zwei abgeschlossene Kammern I und II aus durchsichtigem starrem Material sind durch eine dichte flexible Trennwand t mit einem steifen Mittelteil m verbunden, auf das ein drehbarer Hebel h drückt, auf dem ein Gewicht g von außen verschiebbar angebracht ist. In Kammer I liegt über der Gewichtskonstruktion ein Rost r und auf diesem ein Plasmawasser-Auffangbeutel p, dessen Zulaufschlauch z eingebettet in eine Dichtung durch die Wand der Kammer nach außen führt. In der Kammer II ist ein Begrenzer b enthalten, mit dem von außen die maximale Auslenkung der Trennfläche t begrenzt werden kann. Außerdem führt von der Kammer II ein Druckschlauch d zu einer in der Höhe verstellbaren Nebenkammer III, die einen Klappdeckel k besitzt, mit dessen Hilfe sie dicht geschlossen werden kann. In dieser Nebenkammer ist ein gefüllter Substitutionslösungs-Beutel s eingehängt, wobei der Anschlußschlauch a ungequetscht durch eine Dichtung nach außen führt. Kammer I und II sind mit einer hydraulischen Flüssigkeit — z. B. Wasser — gefüllt.

Zur Vorbereitung der Behandlung wird zunächst ein gefüllter Beutel s mit Substitutionslösung in die Kammer III gebracht und diese dicht verschlossen. Sodann werden die Kammern I, II und III (durch nicht gezeichnete Ventile und Verbindungen) entlüftet, wobei aus einer oben angeordneten Kammer IV die benötigte Flüssigkeit nachläuft und den Plasmawasser-Beutel p entleert, während Ventil v und Ablauf a des Gerätes noch geschlossen sind. Die Kammer III befindet sich zu diesem Zeitpunkt etwa in gleicher Höhe wie Kammer II, z. B. auf einer gemeinsamen Tischplatte. Dann werden die zum Füllen benötigten Verbindungen zu Kammer IV gesperrt und das Ventil v geöffnet.

Das Gewicht g übt über die Trennfläche t auf die Flüssigkeit in Kammer II einen Überdruck aus, während in I ein entsprechender Unterdruck herrscht. Auf einem Differenzdruck-Manometer i kann diese Druckdifferenz direkt abgelesen werden. Sie kann durch Verschieben des Gewichtes g auf dem Hebel h (durch eine nicht gezeigte Spindel) auf den therapeutisch gewünschten Wert gebracht werden.

Nachdem auch der Patient an Hämofilter und Heparinpumpe angeschlossen ist, können der Plasmawasserabgang des Filters an den Zulaufschlauch z und der Restblut führenden Schenkel des Filters an den Auslaßschlauch a luftfrei angeschlossen werden. Nach Öffnen des Ventils v kann sich nun der Überdruck aus Kammer II in Kammer III fortpflanzen und dort genauso viel Substitutionslösung aus dem Beutel s austreiben, wie Plasmawasser aus dem Hämofilter in den Plasmaauffangbeutel p durch den Unterdruck in Kammer I eingesogen wird. Dieser quantitative Austausch von Plasmawasser gegen Substitutionslösung wird volumenkontrolliert beendet, wenn entweder die Substitutionslösung verbraucht oder der Begrenzer b in Kammer II die Bewegung der Trennmembran t begrenzt. Die Dauer des Austauschvorganges kann durch verschieben des Gewichtes g beeinflußt werden.

Zur Entwässerung des Patienten wird zwar das gesamte Plasmawasser auch in den Plasmaauffangbeutel p eingesogen, jedoch ein vorwählbarer Teil der dadurch aus Kammer II verdrängten Flüssigkeit über ein Regelventil w in eine Entwässerungskammer IV abgeleitet. Diese ist mit einer gleichartigen Trennfläche, wie sie die Kammern I und II trennt, gegen die Außenluft abgeschlossen. Ein Begrenzer b wie in Kammer II erlaubt die Bewegung der Trennfläche (und damit die Volumenzunahme der Kammer IV) auf das therapeutisch gewünschte Entwässerungs-Volumen zu begrenzen. Die gewünschte Entwässerungs-Geschwindigkeit wird mit dem Regelventil w eingestellt.

Nach Ende der Behandlung werden die Verbindungen zum Filter gelöst und die Nebenkammer III gegenüber ihrer Lage während der Behandlung angehoben und ihr Deckel geöffnet, bis durch Ausströmen der Flüssigkeit aus Kammer III in Kammer II die Trennfläche t mitsamt dem darauf lastenden Gewicht g in die Ausgangsposition zurückgedrückt ist, wobei gleichzeitig der Plasmaauffangbeutel p entleert wird. Jetzt wird das Ventil v zwischen Kammer II und III geschlossen, Kammer III wieder in die Ausgangslage heruntergeholt und der leere Substitutionslösungs-Beutel s entnommen. Damit ist das Gerät für die nächste Behandlung bereit.

**Patentansprüche**

1. Gerät zur Behandlung urämischer Patienten, bei der das frisch entnommene Blut über ein Hämofilter (F) in Plasmawasser und Restblut getrennt und das Plasmawasser anschließend in eine Austauschvorrichtung geleitet wird, aus dem eine Substitutionslösung nach Zusammenführung mit einem Restblut in den Blutkreislauf zurückgeführt wird, wobei die Austauschvorrichtung aus einem vollständig mit Flüssigkeit gefüllten luftdicht verschließbaren System (A; I, II, III) besteht, welches aus zwei durch eine flexible oder bewegliche Trennfläche (m) voneinander getrennten Kammern (A, B; I, II) besteht, von denen die erste Kammer (A; I) mit Flüssigkeit gefüllt ist und zusätzlich das Plasmawasser aufnehmen kann und die zweite Kammer (B; II) die Substitutionslösung enthält, dadurch gekennzeichnet, daß durch Gewichtsdruck (D; g) auf die flexible oder bewegliche Trennfläche (m) in der ersten Kammer (A; I) ein Unterdruck und in der zweiten Kammer (B, II) ein Überdruck erzeugt wird.

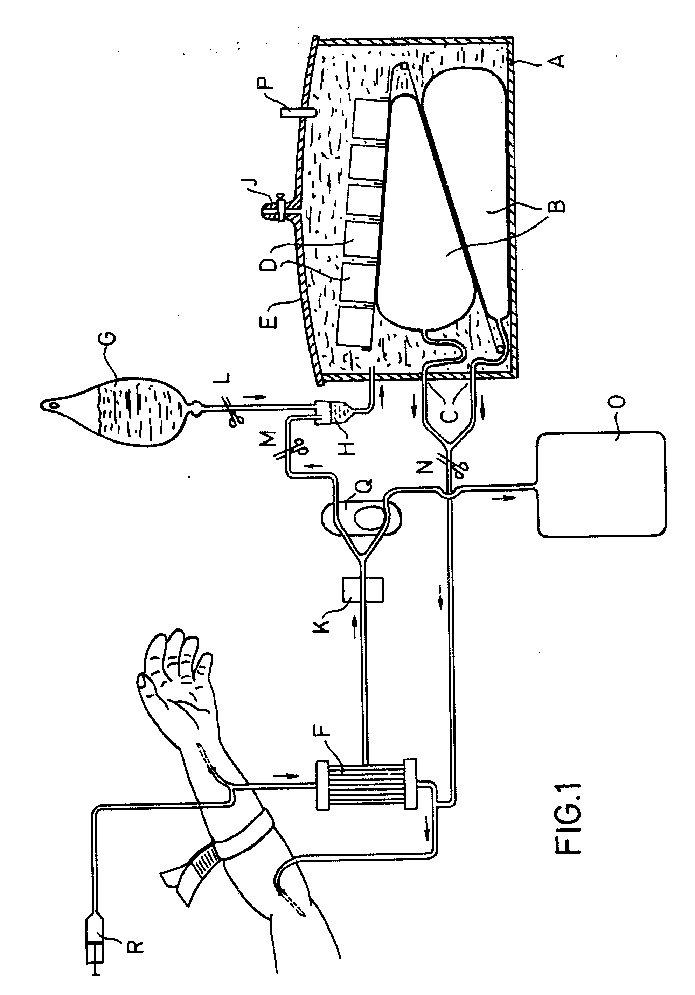

2. Gerät nach Anspruch 1, dadurch gekennzeichnet, daß die zweite Kammer und die flexible Trennfläche durch einen oder mehrere miteinander verbundene Beutel (B) gebildet sind, der bzw. die in der ersten Kammer (A) angeordnet ist bzw. sind und auf den bzw. die die Gewichte (D) direkt einwirken (Fig. 1).

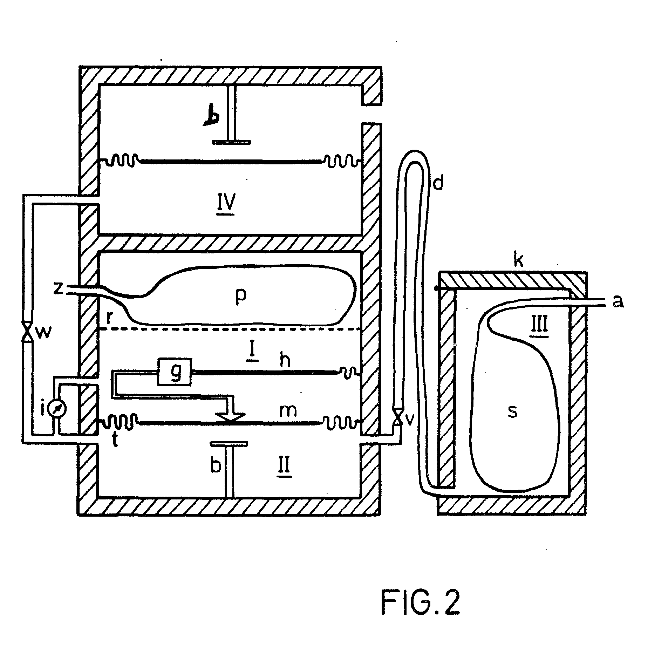

3. Gerät nach Anspruch 1, dadurch gekennzeichnet, daß das Gewicht (g) verschiebbar auf einem Hebel (h) angeordnet ist, der auf die flexible oder bewegliche Trennfläche (m) drückt (Fig. 2).

4. Gerät nach Anspruch 1, dadurch gekennzeichnet, daß in jeder der Kammern, (I, II bzw. III) ein oder mehrere miteinander verbundene Beutel (p, s) zur Aufnahme des Plasmawassers bzw. der Substitutionslösung angeordnet sind und der verbleibende Raum der Kammer mit einer Flüssigkeit gefüllt ist (Fig. 2).

## Claims

1. An apparatus for treating uremic patients, in which the freshly withdrawn blood is separated by means of a hemofilter (F) into plasma water and residual blood, and the plasma water is then passed into an exchange device, from which a substitution solution is returned into the blood circulation after combination with the residual blood, the exchange device consisting of a system (A; I, II, III) which is completely filled with fluid, can be chlosed air-tight and comprises two chambres (A, B; I, II), separated from one another by a flexible or movable partition (m), of which the firste chamber (A; I) is filled with fluid and can additionally take up the plasma water and the second chamber (B; II) contains the substitution solution, characterized in that a subatmospheric pressure is produced in the first chamber (A; I) and a superatmospheric pressure in the second chamber (B; II) by exerting pressure (D; g) on the flexible or movable partition (m).

2. An apparatus as claimed in chiaim 1, characterized in that the second chamber and the flexible partition are formed by one or more bags (B), connected to one another, which is/are arranged in the first chamber (A) and on which the weights (D) directly act (Fig. 1).

3. An apparatus as claimed in claim 1, characterized in that the weight (g) is displaceably mounted on a lever (h) which presses on the flexible or movable partition (m) (Fig. 2).

4. An apparatus as claimed in claim 1, characterized that one or more bags (p, s) connected to one another are arranged in each of the chambers (I, II; and III) for taking up the plasma water and the substitution solution respectively, and the remaining space in the chamber is filled with a liquid (Fig. 2).

## Revendications

1. Appareil pour traiter des maldes urémiques, avec lequel le sang fraîchement prélevé est séparé par l'intermédiaire d'un hémofiltre (F) en plasma et sang résiduel, et le plasma est ensuite conduit dans un dispositif échangeur à partir duquel une solution de substitution est ramenée au circuit sanguin après avoir été réunie au sang résiduel, le dispositif échangeur étant constitué par un système (A; I, II, III) pouvant être obturé de façon étanche à l'air et entièrement rempli de liquide, lequel système est constitué par deux chambres (A, B; I, II) séparées l'une de l'autre par une surface de séparation flexible ou mobile (m), la première chambre (A; I) étant remplie de liquide et pouvant recevoir en plus le plasma, et la seconde chambre (B; II) contenant la solution de substitution, caractérisé par le fait que, par la pression d'un poids (D; g) sur la surface de séparation (m) flexible ou mobile, une dépression est produite dans la première chambre (A; I) et une surpression dans la seconde chambre (B; II).

2. Appareil selon la revendication 1, caractérisé par le fait que la seconde chambre et la surface de séparation flexible sont constituées par un ou plusieurs sacs (B) reliés ensemble, qui est (sont) disposé(s) dans la première chambre (A) et sur le(s)quel(s) les poids (D) agissent directement (fig. 1).

3. Appareil selon la revendication 1, caractérisé par le fait que le poids (g) est monté déplaçable sur un levier (h) qui appuie sur la surface de séparation flexible ou mobile (m) (fig. 2).

4. Appareil selon la revendication 1, caractérisé par le fait que dans chacune des chambres (I, II et III) sont disposés un ou plusieurs sacs (p, s) reliés ensemble, pour recevoir le plasma et la solution de substitution, et le volume restant des chambres est rempli d'un liquide (fig. 2).

FIG.1

FIG.2